(19) Europäisches Patentamt / European Patent Office / Office européen des brevets

(11) **EP 4 431 046 A1**

(12) **EUROPEAN PATENT APPLICATION**
published in accordance with Art. 153(4) EPC

(43) Date of publication:
18.09.2024 Bulletin 2024/38

(21) Application number: 22891872.8

(22) Date of filing: 02.11.2022

(51) International Patent Classification (IPC):
*A61B 34/30* (2016.01)

(86) International application number:
PCT/CN2022/129288

(87) International publication number:
WO 2023/083077 (19.05.2023 Gazette 2023/20)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 11.11.2021 CN 202111334158

(71) Applicant: Shenzhen Edge Medical Co., Ltd.
Shenzhen, Guangdong 518000 (CN)

(72) Inventor: YE, Guoqiang
Shenzhen, Guangdong 518000 (CN)

(74) Representative: KIPA AB
Drottninggatan 11
252 21 Helsingborg (SE)

(54) **METHOD FOR MAINTAINING RC POINT UNCHANGED, AND ROBOTIC ARM, DEVICE, ROBOT AND MEDIUM**

(57) A method for keeping a position of a remote center of manipulation (RC point) of a surgical robot constant in a reference coordinate system ($F_0$) is provided in the present application. The surgical robot (1000) includes: a processor (1001), an adjustment arm (126'), and a cyclone joint (7'). The adjustment arm (126') includes a plurality of joints. The method is executed by a processor and includes the following steps: receiving (S10) a user input for performing adjustment to the cyclone joint; controlling (S20) the cyclone joint to perform angle adjustment according to the user input; calculating (S30) target positions of at least three joints of the adjustment arm according to the angle adjustment performed by the cyclone joint; and controlling (S40) the at least three joints of the adjustment arm to perform position adjustment according to the target positions of the at least three joints so as to maintain the position of the RC point in the reference coordinate system constant. A robotic arm, a slave operating device, a surgical robot, and a computer-readable storage medium are further provided. The present application can realize that a point of contact between a long shaft of a surgical instrument and a minimally invasive incision on a patient remains stationary so as to avoid tearing of the patient's wound.

| | |
|---|---|
| receiving a user input for performing adjustment to the cyclone joint | S10 |
| controlling the cyclone joint to perform angle adjustment according to the user input | S20 |
| calculating target positions of joints of the adjustment arm according to the angle adjustment performed by the cyclone joint | S30 |
| controlling at least three joints of the adjustment arm to perform position adjustment according to the target positions of the joints so as to maintain the position of the RC point in the reference coordinate system constant | S40 |

FIG. 11

**Description**

The present application claims the priority of the Chinese patent application with the application number 202111334158.9 and the application title "METHOD FOR KEEPING RC POINT CONSTANT, ROBOTIC ARM, DEVICE, ROBOT, AND MEDIUM" filed with the CNIPA on Nov. 11, 2021, the entire contents of which are incorporated herein by reference.

TECHNICAL FIELD

The present application relates to the technical field of medical devices, more particularly to a method for keeping a position of an RC point in a reference coordinate system constant, a robotic arm, a slave operating device, a surgical robot, and a computer-readable storage medium.

BACKGROUND

Minimally invasive surgery refers to a surgical method that uses modern medical instruments such as a laparoscopy and a thoracoscopy and related instruments to perform surgery inside a human cavity. Compared with traditional surgical methods, minimally invasive surgery has the advantages of smaller trauma, fewer pain, and faster recovery.

With the development of science and technology, robotic technology for minimally invasive surgery has been gradually matured and widely used. A minimally invasive surgical robot usually includes a master operating console and a slave operating device. The master operating console is configured to send control commands to the slave operating device according to the doctor's operation to control the slave operating device. The slave operating device is configured to perform corresponding surgical operations in response to the control commands sent by the master operating console. A surgical instrument is in connection with a driving device of the slave operating device for performing a surgical operation, and the surgical instrument has a long shaft and an end effector located at an end of the long shaft. Theoretically, in the process of the surgery, a point of contact between the long shaft of the surgical instrument and the minimally invasive incision on the patient should remain stationary to avoid tearing of the patient's wound.

However, the existing technology cannot ensure that such point of contact remains stationary at the patient's minimally invasive incision.

SUMMARY

A main object of the present application is to provide a method for keeping a position of a RC point of a surgical robot constant in a reference coordinate system, a robotic arm, a slave operating device, a surgical robot, and a computer-readable storage medium, which aim at realizing that a point of contact between a long shaft of a surgical instrument and a minimally invasive incision on a patient remains stationary so as to avoid tearing of the patient's wound.

In order to achieve the above object, the present application provides a method for keeping a position of a remote center of manipulation (RC point) of a surgical robot constant in a reference coordinate system ($F_0$). The surgical robot comprise: a processor, an adjustment arm, and a cyclone joint. The adjustment arm comprises a plurality of joints. The method is executed by a processor and comprises the following steps:

receiving a user input for performing adjustment to the cyclone joint;
controlling the cyclone joint to perform angle adjustment according to the user input;
calculating target positions of at least three joints of the adjustment arm according to the angle adjustment performed by the cyclone joint; and
controlling the at least three joints of the adjustment arm to perform position adjustment according to the target positions of the at least three joints so as to maintain the position of the RC point in the reference coordinate system constant.

Optionally, the step of calculating the target positions of the at least three joints of the adjustment arm according to the angle adjustment performed by the cyclone joint comprises:

obtaining the position of the RC point in the reference coordinate system ($F_0$) and a constant transformation ($T_{0a}$) from the reference coordinate system ($F_0$) to an adjustment arm coordinate system ($F_a$);
obtaining an angle value of the cyclone joint;
calculating a first transformation ($T_{bc}$) from a cyclone joint coordinate system ($F_b$) to an RC point coordinate system ($F_c$) based on the angle value of the cyclone joint;
calculating a second transformation ($T_{ab}$) from the adjustment arm coordinate system ($F_a$) to the cyclone joint coordinate system ($F_b$) based on the position of the RC point in the reference coordinate system ($F_0$), the constant transformation ($T_{0a}$), and the first transformation ($T_{bc}$); and
calculating the target positions of the at least three joints of the adjustment arm based on the second transformation ($T_{ab}$).

Optionally, the step of obtaining the position of the RC point in the reference coordinate system ($F_0$) comprises:

obtaining a third transformation ($T_{0c}$) from the reference coordinate system ($F_0$) to the RC point coordinate system ($F_c$) through a kinematic calculation; and

acquiring the position of the RC point in the reference coordinate system ($F_0$) according to the third transformation ($T_{0c}$).

Optionally, the step of obtaining the angle value of the cyclone joint comprises:
obtaining the angle value of the cyclone joint from a joint encoder.

The joint encoder comprises a position sensor, and the position sensor is configured to measure an angle value of a joint where the joint encoder is located.

Optionally, the step of calculating the first transformation ($T_{bc}$) from the cyclone joint coordinate system ($F_b$) to the RC point coordinate system ($F_c$) comprises:
determining the first transformation ($T_{bc}$) from the cyclone joint coordinate system ($F_b$) to the RC point coordinate system ($F_c$) based on the angle value of the cyclone joint.

Optionally, the step of calculating the second transformation ($T_{ab}$) from the adjustment arm coordinate system ($F_a$) to the cyclone joint coordinate system ($F_b$) comprises:

constructing a calculation model for the position of the RC point in the reference coordinate system ($F_0$) based on a coordinate transformation relationship; and
expanding the calculation model along an X-direction, a Y-direction, and a Z-direction to obtain three equations.

The equations comprise a multivariate linear equation system, in which the target positions of the at least three joints of the adjustment arm are used as unknown variables.

Optionally, the step of constructing the calculation model for the position of the RC point in the reference coordinate system ($F_0$) based on the coordinate transformation relationship comprises:

$$P_{0c} = R_{0a} * R_{ab} * P_{bc} + R_{0a} * P_{ab} + P_{0a},$$

in which,

$P_{0c}$ represents a position component of a transformation $T_{0c}$; $R_{0a}$ represents an attitude component of the transformation $T_{0a}$; $P_{0a}$ represents a position component of the transformation $T_{0a}$; $R_{ab}$ represents an attitude component of the transformation $T_{ab}$; $P_{ab}$ represents a position component of the transformation $T_{ab}$; $R_{bc}$ represents an attitude component of the transformation $T_{bc}$; and $P_{bc}$ represents a position component of the transformation $T_{bc}$.

Optionally, the step of expanding the calculation model along the X-direction, the Y-direction, and the Z-direction to obtain the three equations comprises:

expanding equation $P_{0c} = R_{0a} * R_{ab} * P_{bc} + R_{0a} * P_{ab} + P_{0a}$ according to position components in the X-direction, the Y-direction, and the Z-direction, respectively, to obtain a compensation solution model comprising the following equation system:

$$\begin{cases} P_x = f_1(\theta_1, \theta_2 \cdots \theta_i) \\ P_y = f_2(\theta_1, \theta_2 \cdots \theta_i) \\ P_z = f_3(\theta_1, \theta_2 \cdots \theta_i) \end{cases};$$

in which, $P_x$, $P_y$, and $P_z$ are three components of the RC point position $P_{0c}$ in the X-direction, the Y-direction, and the Z-direction, respectively, and $f_1$, $f_2$, and $f_3$ represent corresponding calculation functions, which are all related to positions $(\theta_1, \theta_2 \dots \theta_i)$ of the at least three joints of the adjustment arm.

Optionally, the step of calculating target positions of at least three joints of the adjustment arm comprises: calculating the target positions of the at least three joints of the adjustment arm according to the compensation solution model.

Optionally, the adjustment arm comprises: a first rotary joint, a first linear joint, a second rotary joint, and a second linear joint; or alternatively, the adjustment arm comprises: the first linear joint, the second rotary joint, and the second linear joint.

Optionally, a position $\theta_1$ of the first rotary joint is a constant, and target positions $(\theta_2, \theta_3, \theta_4)$ of the first linear joint, the second rotary joint, and the first linear joint are obtained according to the following compensation solution model:

$$\begin{cases} P_x = f_1(\theta_1, \theta_2, \theta_3, \theta_4) \\ P_y = f_2(\theta_1, \theta_2, \theta_3, \theta_4) \\ P_z = f_3(\theta_1, \theta_2, \theta_3, \theta_4) \end{cases}.$$

Optionally, in order to achieve the above object, the present application further provides a robotic arm, which is in connection with an orientation platform. The robotic arm comprises:

an adjustment arm, in connection with the orientation platform, the adjustment arm comprising a plurality of joints;
a cyclone joint, in connection with the adjustment arm, with an axis passing through the cyclone joint being defined as a cyclone axis; and
a processor, in connection with the cyclone joint and the adjustment arm.

The processor is configured to execute the method as described in the above.

In order to achieve the above object, the present application further provides a slave operating device. The slave operating device comprises an orientation platform. The slave operating device comprises the robotic arm as described in the above, and the robotic arm is in connection with the orientation platform.

In order to achieve the above object, the present application further provides a surgical robot. The surgical robot comprises: the slave operating device as described in the above; and a master operating device, configured to control the slave operating device.

In order to achieve the above object, the present application further provides a computer-readable storage medium. The computer-readable storage medium is stored with a program for keeping a position of an RC point in a reference coordinate system ($F_0$) constant, which, when being executed by a processor, causes the processor to implement steps of the method for keeping the position of the RC point in the reference coordinate system ($F_0$) constant as described in the above.

In the method for keeping the position of the RC point in the reference coordinate system constant, the robotic arm, the slave operating device, the surgical robot, and the computer-readable storage medium provided by the present application, the user input is received; the cyclone joint is controlled to perform angle adjustment according to the user input; then target positions of at least three joints of the adjustment arm are calculated according to the angle adjustment performed by the cyclone joint; after that, the at least three joints of the adjustment arm are controlled to perform position adjustment according to the target positions of the at least three joints so as to maintain the position of the RC point in the reference coordinate system constant. In this way, the point of contact between the long shaft of the surgical instrument and the minimally invasive incision on the patient remains stationary, thereby avoiding tearing of the patient's wound.

DETAILED DESCRIPTION OF THE DRAWINGS

FIG. 1 is a schematic structural view of a surgical robot according to an embodiment of the present application;
FIG. 2 is a schematic structural view of a master operating console in FIG. 1 according to an embodiment of the present application;
FIG. 3 is a schematic structural view of an electronic device trolley in FIG. 1 according to an embodiment of the present application;
FIG. 4 is a block schematic structural diagram of FIG. 1;
FIG. 5 is a simplified schematic structural view of a slave operating device in FIG. 1 according to an embodiment of the present application;
FIG. 6 is a simplified structural schematic view of a slave operating device in FIG. 1 according to another embodiment of the present application;
FIG. 7 is a simplified structural schematic view of a slave operating device according to an embodiment of the present application;
FIG. 8 is a simplified structural schematic view of a robotic arm according to an embodiment of the present application;
FIG. 9 is a schematic structural view of a surgical instrument in FIG. 5 according to an embodiment of the present application;
FIG. 10 is a schematic structural view of an operating arm and an adjustment arm according to an embodiment of the present application;
FIG. 11 is a schematic flow chart of a method for keeping a position of an RC point in a reference coordinate system ($F_0$) constant according to an embodiment of the application;
FIG. 12 is a schematic flow chart of an adjustment arm performing compensation movement according to an embodiment of the present application; and
FIG. 13 is a schematic diagram of a hardware structure of a robotic arm, a slave operating device, or a surgical robot according to an embodiment of the present application.

The realization, functional features, and advantages of the present application will be further described with reference to the accompanying drawings in conjunction with the embodiments.

DETAILED DESCRIPTION OF THE EMBODIMENTS

The following will clearly and completely explain the technical solutions in the embodiments of the present application with reference to the accompanying drawings in the embodiments of the present application. Obviously, the described embodiments are only part of, rather than all of, the embodiments of the present application. Based on the embodiments in the present application, all other embodiments obtained by those skills in the art, without paying creative efforts, fall within the protection scope of the present application.

It should be noted that all directional indications (such as upper, lower, left, right, front, rear, etc.) in embodiments of the present application are only used to explain the relative positional relationship, movement conditions, etc. among components in a particular posture (as shown in the figures). If the specific posture changes, the directional indication may also change accordingly.

In the present application, unless otherwise clearly specified and limited, the terms "connection" and "fixation" should be interpreted in a broad sense, for example, "fixation" can be a fixed connection, a detachable connection, or integrated as a whole, can be a mechanical connection or an electrical connection; can be a direct connection or an indirect connection through an intermediate medium, and can be an internal communication between two elements or an interaction relationship between two elements, unless otherwise clearly defined.

The specific meanings of the above terms in present application can be understood by those skilled in the art according to specific situations.

In addition, descriptions such as "first", "second" and the like in present application are only for description purposes, and should not be understood as indicating or implying their relative importance or implicitly indicating the number of indicated technical features. Thus, the features defined as "first" and "second" may explicitly or implicitly include at least one of these features. In addition, the technical solutions of the various embodiments can be combined with each other, on the premise that those skilled in the art are able to realize such combination of technical solutions. When the combination of technical solutions is contradictory or cannot be realized, it should be considered that the combination of technical solutions does not exist, nor is such combination of technical solutions within the scope of protection as claimed in the present application.

The term "instrument" is used herein to describe medical equipment intended for insertion into a patient's body and for performing surgical or diagnostic procedures, the instrument including an end effector, which may be a surgical instrument, such as an electrocautery, a clamp, a stapler, a cutter, an imaging device, such as an endoscope or an ultrasound probes, and the like. Some instruments used in embodiments of the present application further include an articulation member (such as a joint assembly) for the end effector, so that the position and orientation of the end effector can be manipulated and moved in one or more mechanical degrees of freedom relative to an instrument axis. Further, the end effector includes functional mechanical degrees of freedom, such as opening and closing jaws. The instrument may further include stored information that may be updated by the surgical system, whereby the memory system may provide one-way or two-way communication between the instrument and one or more system components.

Unless otherwise defined, all technical and scientific terms used herein have the same meaning as commonly understood by those skilled in the art which the present application belongs to. The terminology used herein in the description of the application is only for the purpose of describing specific embodiments, and is not intended to limit the application. As used herein, the terms "and/or" include any and all combinations of one or more of the associated listed items.

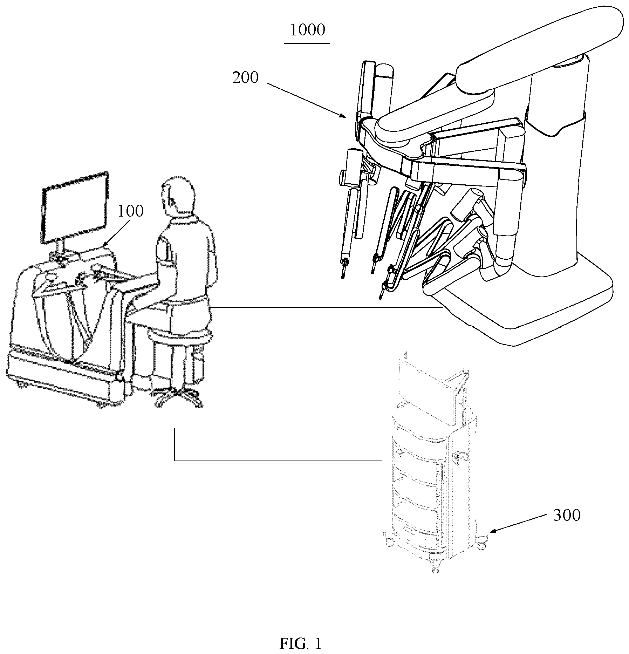

As shown in FIG. 1, a surgical robot 1000 may be configured to perform minimally invasive diagnostic or surgical procedures on a patient lying flat on an operating table. The surgical robot 1000 includes a master operating console 100 and a slave operating device 200. The master operating console 100 is configured to send control commands to the slave operating device 200 according to the doctor's operation to control the slave operating device 200. The slave operating device 200 is configured to perform corresponding surgical operations in response to the control commands sent by the master operating console 100. The surgical robot may further include an electronic device trolley 300 electrically in connection with the master operating console 100. The surgeon can observe a surgical site through the master operating console 100, and the slave operating device 200 can manipulate at least one removably connected surgical instrument (not shown) through the minimally invasive incision on the patient. The image of the surgical site can be obtained by an endoscope (not shown in the figure), such as a stereoscopic endoscope, which can be manipulated by the slave operating device 200 to orient the endoscope. The electronic device trolley 300 may be configured to display an image of the surgical site, so as to display to a physician's assistant.

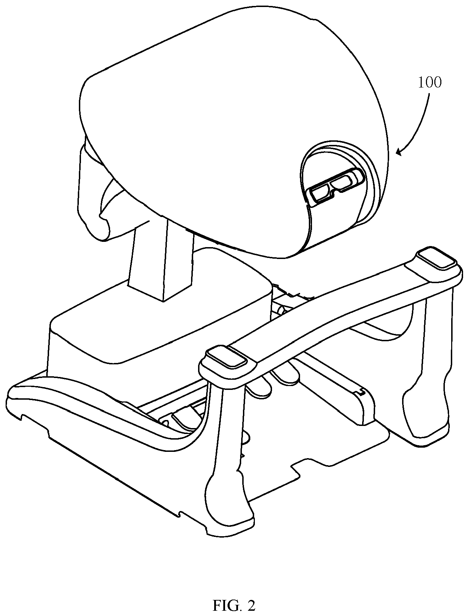

FIG. 2 is a schematic diagram of a master operating console 100 for a surgeon. The master operating console 100 for the surgeon includes: a left-eye display and a right-eye display (not shown in the figure), which are configured to present to the surgeon a stereoscopic view capable of depth perception of the surgical site. The master operating console 100 further includes one or more input control devices (not shown in the figure), and the surgeon operates the input control devices to make the slave operating device 100 manipulate one or more surgical instruments synchronously. The input control device can provide the same degrees of freedom as the surgical instruments associated thereto, thereby providing the surgeon with telepresence, or provide the perception that the input control device is integrated with the surgical instruments, so that the surgeon has a strong sense of direct control of the surgical instruments.

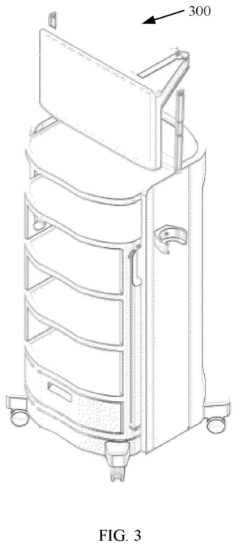

FIG. 3 is a schematic diagram of an electronic device trolley 300. The electronic device trolley 300 can be in connection with the endoscope and may include a processor for processing the captured image, and the captured image is used to be displayed to the surgeon at the surgeon's console or at another suitable computer located locally and/or remotely. For example, in the case of a stereoscopic endoscope, an image host in the electronic device trolley 300 may process the captured image to present a coordinated stereoscopic image of the surgical site to the surgeon. Such coordination may include alignment between opposing images, and may include adjusting the stereoscopic working distance of the stereoscopic endoscope. As another example, image processing may include using predetermined camera calibration parameters to compensate for imaging errors of the image capture device, such as optical aberrations.

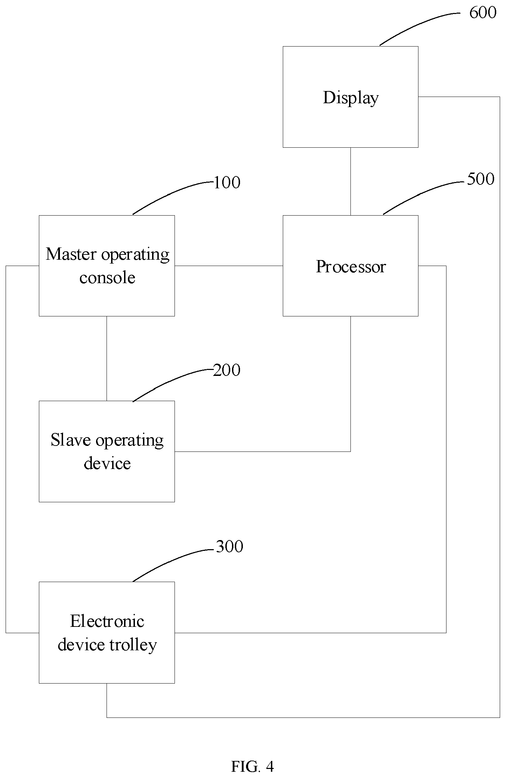

As shown in FIG. 4, the master operating console 100 for the surgeon may be used by the surgeon to control the slave operating device 200 during minimally invasive surgery. The slave operating device 200 may capture an image of a surgical site using an imaging device such as a stereoscopic endoscope and output the captured image to the master operating console 100 and the electronic device trolley 300. As discussed in the above, the electronic device trolley 300 is able to process the captured image in various ways prior to any subsequent display. For example, the electronic device trolley 300 may overlay the captured image with a virtual control interface before displaying the combined image to the surgeon via the surgeon's master operating console 100. The captured image may be output from the slave operating device 200 for processing outside the electronic device trolley 300. For example, the captured image can be output from the slave operating device 200 to the processor 500, and the processor 500 may be configured to process the captured image. The image may also be processed by a combination of the electronic device trolley 300 and the processor 500, and the electronic device trolley 300 and the processor 500 may be coupled together for joint, sequential, and/or combined processing of the captured image. One or more separate displays 600 may also be coupled to the processor 500 and/or the electronic device trolley 300 for local and/or remote display of the image, such as an image of the surgical site or other relevant images. It can be understood that the processor may include a processor of the master operating console 100, a processor of the slave operating device 200, and a processor of the image host (not shown in the figure) on the electronic device trolley 300. For ease of understanding, only one processor is identified here.

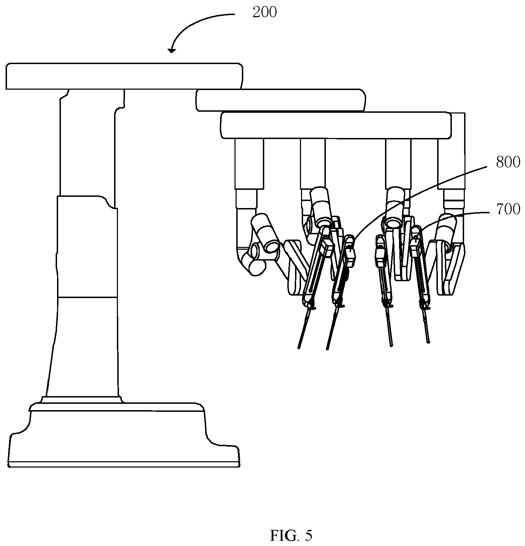

As shown in FIG. 5, the slave operating device 200 provides manipulation of three surgical instruments 700 and imaging devices 800, the imaging devices 800 may be such as stereoscopic endoscopes for capturing images of a surgical site. The imaging devices 800 and the surgical instruments 700 can be arranged and manipulated at the incision on the patient in such a manner that the kinematic RC point (remote center of manipulation) is maintained at the incision, thereby minimizing the size of the incision. The images of the surgical site may include images of distal ends of the surgical instruments 700 when the distal ends of the surgical instruments 700 are disposed within the field of views of the imaging devices 800.

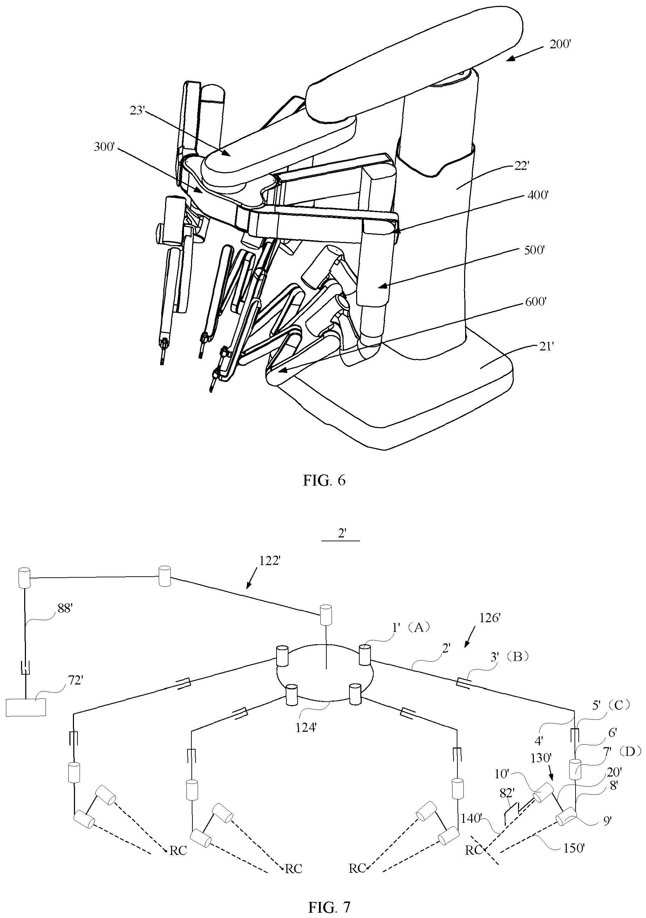

As shown in FIG. 6, the slave operating device 200 includes a base 200', an orientation platform 300' installed on the base 200', and a robotic arm 400' in connection with the orientation platform 300'. The base 200' may further include: a base body 21', a column 22' disposed on the base body 21', a suspension arm 23' in connection with the column 22', and an orientation platform 300' is in connection with the suspension arm 23'. The robotic arm 400' includes: an adjustment arm 500' in connection with the orientation platform 300', an operating arm 600' in connection with the adjustment arm 500', and a surgical instrument (not shown in the figure) installed on the operating arm. The surgical instrument may be an electric cauterizer, a clamp, a stapler, a scissor, etc., for performing surgical operations, and may also be a camera or other surgical instruments for acquiring images. Multiple surgical instruments are inserted into the body of the patient via different incisions.

As shown in FIGS. 7-8, the orientation platform 124' is in connection with the suspension arm 122', the adjustment arm 126' is in connection with the orientation platform 124', the operating arm 130' is attached to the adjustment arm 126', and is supported by the adjustment arm 126'. The adjustment arm 126' may include: a rotary joint 1', a rotary arm 2', a linear joint 3', a translation arm 4', a linear joint 5', a lifting arm 6', a rotary joint 7', a rotary arm 8', a cyclone Joint 9', a deflection joint 10'. The components of the adjustment arm 126' are connected in sequence.

The adjustment arm 126' is in rotatable connection with the orientation platform 124' through the rotary joint 1', and is supported by the orientation platform 124'. The orientation platform 124' is in rotatable connection with and supported by the suspension arm 122'. The suspension arm 122' is fixedly attached to and supported by the base body 72' via the column 88'. The suspension arm 122' is operable to selectively set the angle of the orientation platform 124' relative to the base 72'. The adjustment arm 126' is operable to selectively set the angle of the associated operating arm 130' relative to the orientation platform 124'.

The operating arm 130' further includes a coupling link 20' fixedly connecting the deflection joint 10' to the cyclone joint 9'. The cyclone joint 9' is operable to rotate the deflection joint 10' relative to a support link 128' about a cyclone axis 150'. The cyclone axis 150' does not pass through the RC point. The deflection axis 140' passes through the RC point. Operation of the cyclone joint 9' can be configured to reorient a parallelogram mechanism 82' relative to the patient without moving the RC point relative to the patient.

As shown in FIGS. 7-8, the rotation of the rotary joint 1' drives the rotation of the rotary arm 2', the movement of the linear joint 3' drives the translation arm 4' so that the translation arm 4' can move in a horizontal direction, and the movement of the linear joint 5' drives the lifting arm 6' to enable the lifting arm 6' to move in a vertical direction, the rotation of the rotary joint 7' drives the rotary arm 8' to rotate, and the rotation of the cyclone joint 9 drives the parallelogram mechanism 82' to rotate about the cyclone axis 150'. The linear joint 3' and the linear joint 5' are linked, and the rotary joint 1', the rotary joint 7', and the cyclone joint 9' may rotate independently.

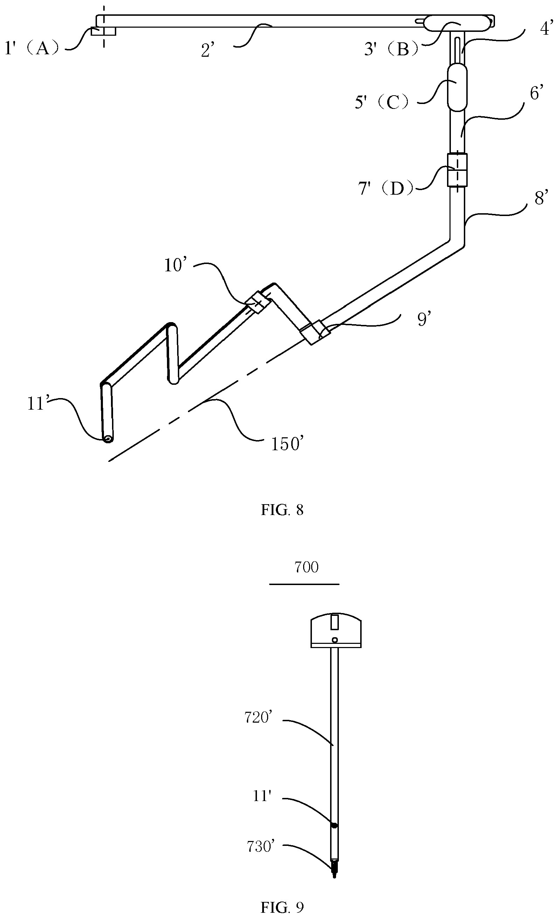

As shown in FIG. 9, the surgical instrument 700 has a long shaft 720' and an end effector 730' located at an end of the long shaft 720', and an RC point (remote center of manipulation) is arranged near a side of the long shaft 720' near the end effector 730'. The RC point may also be referred to as an instrument fixed point 11'. The instrument fixed point 11' refers to that after the surgical instrument is installed on the instrument carrying arm (not shown in the figure) on the operating arm 130', a distal end of the long axis 720' of the surgical instrument remains at a position relatively fixed during the surgery. The cyclone axis 150' of the cyclone joint 9' does not pass through the instrument fixed point 11'. The instrument fixed point 11' coincides with the RC point of the surgical robot.

Before the surgery starts, an end of the instrument carrying arm is dragged to a position close to the surgical site of the patient, and the rotary joint 1', the linear joint 3', the linear joint 5', and the rotary joint 7' are linked to compensate for the deviation of the instrument fixed point 11' which may be caused by the rotation of the cyclone joint 9, thereby keeping the instrument fixed point 11' coincide with the RC point. In other words, through the adjustment and compensation of the adjustment arm 126', the position of the RC point in the coordinate system of the orientation platform may be maintained constant.

In other embodiments, the linear joint 3', the linear joint 5', and the rotary joint 7' are linked, and the rotary joint 1' may not rotate, so as to compensate for the deviation of the instrument fixed point 11' that may be caused by the movement of the cyclone joint 9', and to maintain the position of the RC point in the coordinate system of the orientation platform constant, thereby avoiding tearing of the incision at a surgical instrument entrance.

According to the robot kinematics modeling method, such as a Denavit-Hartenberg (DH) method, the kinematics modeling of the orientation platform, the adjustment arm, and the instrument arm is performed, which includes the definition of the coordinate system and the definition of the coordinate transformation relationship. In the present application, transformation refers to the transformation of a transformation matrix or a coordinate system.

Combining with the coordinate transformation calculation and the kinematic calculation, the compensation movement of the adjustment arm during the adjustment of the movement of the cyclone joint is realized, and the kinematic calculation includes a forward solution calculation and an inverse solution calculation.

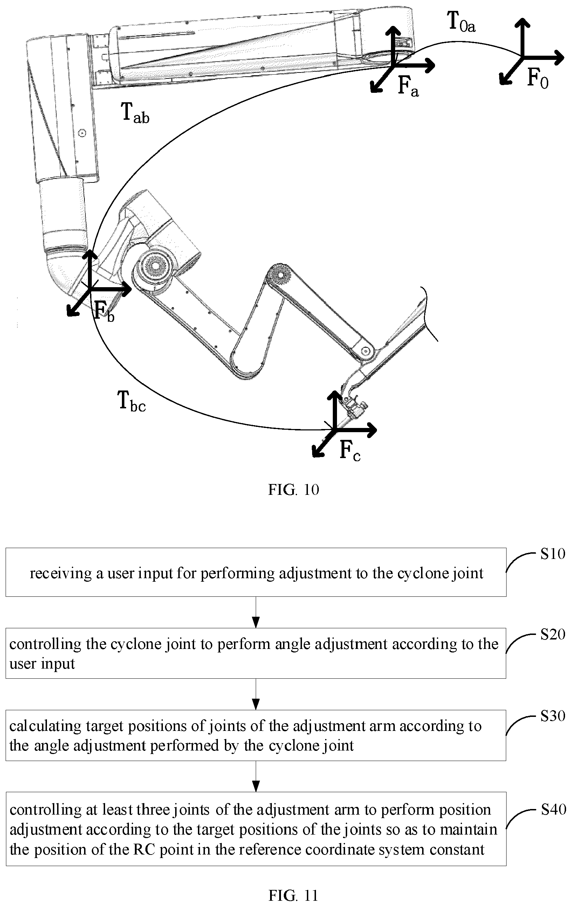

As shown in FIG. 10, a reference coordinate system $F_0$ of an orientation platform, an adjustment arm coordinate system $F_a$, a cyclone joint coordinate system $F_b$, and an RC point coordinate system $F_c$ are defined.

The transformation from the reference coordinate system $F_0$ of the orientation platform to the adjustment arm coordinate system $F_a$ is a constant transformation $T_{0a}$. The transformation from the adjustment arm coordinate system $F_a$ to the cyclone joint coordinate system $F_b$ is determined by positions of various joints of the adjustment arm and is defined as $T_{ab}$. The transformation from the cyclone joint coordinate system $F_b$ to the RC point coordinate system $F_c$ is determined by an angle value of the cyclone joint and is defined as $T_{bc}$.

When the user adjusts the cyclone joint, the cyclone joint performs motion adjustment according to the user input, and the adjustment arm joint performs compensation motion based on kinematic calculation, such that the position of the RC point in the coordinate system of the orientation platform remains constant.

As shown in FIG. 11, in an embodiment, the present application provides a method for keeping a position of a remote center of manipulation (RC point) of a surgical robot constant in a reference coordinate system ($F_0$). The method is carried out by the following steps:

In step S10, a user input configured to perform the adjustment of the cyclone joint is received;

In this embodiment, buttons are correspondingly arranged on the operating arm, for example, button 1 and button 2 are arranged near a top of an instrument carrying arm. When the user clicks or presses the button 1, the operating arm will rotate clockwise around the cyclone axis, and when the user clicks or presses the button 2, the operating arm will rotate counterclockwise around the cyclone axis, in such condition, an angle of the cyclone joint will be adjusted.

In step S20, the cyclone joint is controlled to perform angle adjustment according to the user input;

In step S30, target positions of at least three joints of the adjustment arm are calculated according to the angle adjustment performed by the cyclone joint; and

In step S40, the at least three joints of the adjustment arm are controlled to perform position adjustment according to the target positions of the at least three joints so as to maintain the position of the RC point in the reference coordinate system constant.

The details will be introduced below and will not be repeated here.

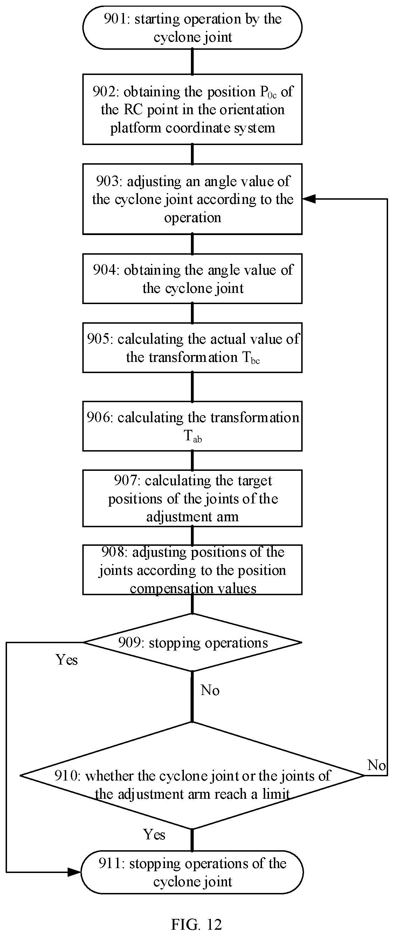

As shown in FIG. 12, the specific compensation method of the adjustment arm is performed as follows: In step 901: the operation of the cyclone joint is started, in which, based on the user input, the processor responds to the user input and starts to execute the adjustment of the cyclone joint.

Herein, the processor may be a robotic arm processor.

In step 902: the position $P_{0c}$ of the RC point in the reference coordinate system is obtained.

Through the kinematics forward solution calculation, that is, through the coordinate transformation relationship, the transformation $T_{0c}$ from the reference coordinate system $F_0$ of the orientation platform to the RC point coordinate system $F_c$ is obtained:

$$T_{0c}=T_{0a}*T_{ab}*T_{bc};$$

The transformation matrix can be expressed as $T=\begin{bmatrix} R & P \\ 0 & 1 \end{bmatrix}$, in which, R is an attitude component, and P is a position component.

The position component in this transformation is the position $P_{0c}$ of the RC point in the reference coordinate system, and the processor stores the position $P_{0c}$, and the purpose of the subsequent compensation method is to keep the position $P_{0c}$ constant.

In step 903: an angle value of the cyclone joint is adjusted according to the operation. Generally speaking, based on user input, the cyclone joint will perform angle adjustment according to a specific motion mode (such as JOG motion). The user's input can be that the user presses the buttons for adjusting the cyclone join which are arranged on the operating arm.

In step 904: the angle value of the cyclone joint is obtained. The angle value of the cyclone joint can be obtained from the joint encoder. The joint encoder may be a position sensor, which is installed at the joint and can measure the angle value of the joint.

In step 905: the value of the transformation $T_{bc}$ is calculated. As mentioned above, the transformation from the cyclone joint coordinate system $F_b$ to the RC point coordinate system $F_c$ is determined by the angle value of the cyclone joint. Based on the angle value of the cyclone joint, an actual value of the transformation $T_{bc}$ may be obtained through a link transformation relationship.

The link transformation relationship in the present application is obtained through a general series DH modeling method. This term appears elsewhere in the present application will not be repeated again.

In step 906: the transformation $T_{ab}$ is calculated.

The above is to calculate the compensation value, and the method of constructing the compensation solution model is as follows:
The position component in $T_{0c}=T_{0a}*T_{ab}*T_{bc}$ is decomposed to get:

$$P_{0c}=R_{0a}*R_{ab}*P_{bc}+R_{0a}*P_{ab}+P_{0a}, i$$

in which,

$P_{0c}$ represents a position component of a transformation $T_{0c}$; $R_{0a}$ represents an attitude component of the transformation $T_{0a}$; $P_{0a}$ represents a position component of the transformation $T_{0a}$; $R_{ab}$ represents an attitude component of the transformation $T_{ab}$; $P_{ab}$ represents a position component of the transformation $T_{ab}$; $R_{bc}$ represents an attitude component of the transformation $T_{bc}$; and $P_{bc}$ represents a position component of the transformation $T_{bc}$.

$P_{0c}$ has been recorded as mentioned in the above. $R_{0a}$ and $P_{0a}$ represent the relative attitude and position between the reference coordinate system $F_0$ of the orientation platform and the adjustment arm coordinate system $F_a$, both of which are fixed parameters and are known. $R_{bc}$ and $P_{bc}$ represent the relative attitude and position between the cyclone joint coordinate system $F_b$ and RC point coordinate system $F_c$. In the adjustment process of the cyclone joint, only the angle value of the cyclone joint is a variable, which can be obtained from the previous step. It can be seen that in the above equation, only

$R_{ab}$ and $P_{ab}$ are unknown variables, which are related to the positions of the joints of the adjustment arm.

When the above equation is expanded according to position components in the X-direction, the Y-direction, and the Z-direction, respectively, a compensation solution model comprising the following equation system are obtained:

$$\begin{cases} P_x = f_1(\theta_1, \theta_2 \cdots \theta_i) \\ P_y = f_2(\theta_1, \theta_2 \cdots \theta_i) \, ; \\ P_z = f_3(\theta_1, \theta_2 \cdots \theta_i) \end{cases}$$

in which, $P_x$, $P_y$, and $P_z$ are three components of the RC point position $P_{0c}$ in the X-direction, the Y-direction, and the Z-direction, respectively, and $f_1$, $f_2$, and $f_3$ represent corresponding calculation functions, which are all related to positions $(\theta_1, \theta_2 \ldots \theta_i)$ of the at least three joints of the adjustment arm.

In step 907: the target positions of the joints of the adjustment arm are calculated.

According to the solution model mentioned above, if the number of joints of the adjustment arm is 3, the target position $(\theta_1, \theta_2 \cdots \theta_i)$ of the adjustment arm can be obtained by solving the equation system; if the number of joints of the adjustment arm is greater than 3, the number of equations is smaller than the number of the solutions to be solved, which means that the corresponding adjustment arm has redundant joints, in such condition, a solution strategy needs to be defined.

Specifically, as shown in FIG. 8, the adjustment arm may have four joints, which are the first joint A, the second joint B, the third joint C, and the fourth joint D from top to bottom, respectively. For example, from top to bottom are a rotary joint A, a linear joint B, a linear joint C, and a rotary joint D.

After the cyclone joint moves, it is necessary to consider the movement compensation in the X-direction, the Y-direction, and the Z-direction. Combined with the structural characteristics, the optional compensation combinations of the adjustment arm include the following four combinations:
combination 1): A, B, and C; combination 2) A, C, and D; combination 3) B, C, and D; and combination 4) A, B, C, and D.

Explanation 1: if the combination involves joints A, B, and D, since these three joints are either horizontal rotation joints or horizontal translation joints, such combination cannot realize vertical compensation.

Explanation 2: if the combination is the combination 1) or combination 2) in the above four combinations, when the RC point coincides with or is close to an axis of the first rotary joint, the mobility of the rotary joint A in the combination 1) and the combination 2) is pathological or close-to-pathological, which specifically has the following performances: in a case where the RC point is close to coincide with the axis of the first rotary joint, the rotary joint A needs to move a large range in order to achieve the position compensation; and in a case where the RC point coincides with the axis of the first rotary joint, the rotary joint A fails and is unable to realize the compensation.

In the above steps, the term "mobility" refers to the feasibility and effectiveness of the robotic arm to adjust the movement of the end through the movements of the various joints. The term "pathological" refers to that in some states, the combined motion of the various joints of the robotic arm cannot achieve a desired movement of the end.

The term "close-to-pathological" refers to that the combined motion of various joints of the robotic arm requires a higher speed to meet the desired movement of the end. The specific performance is as described in the above.

The optimal compensation combination in the above four combinations are combination 3) or combination 4).

For the combination 3), as mentioned in the above, $\theta_1$ is a known variable, which represents a current position of the first rotary joint. In such condition, the compensation values $(\theta_2, \theta_3, \theta_4)$ can be derived according to the above equation system.

For the combination 4), the joints are redundant, and a redundant strategy needs to be defined when solving the solutions.

The idea of compensation combination of the adjustment arm is as follows: a. a main task of the movement of the adjustment arm is to compensate the deviation of the position of the RC point caused by the movement of the cyclone joint; and b. the spatial positioning of the four robotic arms are to be considered in order to avoid collisions during the operation.

Further, the first rotary joint can be defined as an active joint. When the cyclone joint adjusts the angle value according to the operation, the processor aims to adjust the angle between the first adjustment arm and the second adjustment arm, so as to enable the first joint A to move in the direction of the recommended angle; and the rest joints of the adjustment arm are derived according to the above equation system to obtain the corresponding compensation values $(\theta_2, \theta_3, \theta_4)$.

Four robotic arms are arranged at the orientation platform, and the recommended angle refers to the angle between two adjustment arms. The angle between the first adjustment arm and the second adjustment arm refers to an angle between a connecting rod connecting any two adjacent joints on the first adjustment arm and a connecting rod connecting corresponding two adjacent joints on the second adjustment arm.

In step 908: positions of the joints of the adjustment arm are adjusted according to the position compensation values obtained from the above calculation.

In step 909: the cyclone joint stops operation.

In step 910: if the cyclone joint reaches a limit, or the joints of the adjustment arm reach a limit, step 911 is executed, which is as follows: the operation of the cyclone joint stops, and neither the cyclone joint nor the joints of the adjustment arm move. The limit is a software limit, that is, through the calculation, all joints can reach a state where none of the joints is able to continue to move.

If the cyclone joint and the adjustment arm do not reach the limit, step 903 is executed.

As described in the above embodiments, a method for keeping the position of the RC point of the surgical robot constant in the reference coordinate system during the movement of the cyclone joint is provided. Based on the mechanical structure and compensation algorithm, it can be ensured that the instrument fixed point coincides with the RC point during the preoperative preparation work, such that it can be ensured that the position of the RC point in the reference coordinate system remains constant during the surgery, no matter how the operating arm moves, and the operating arm will never touch the patient. By adjusting the spatial positioning of multiple operating arms, the problem of robotic arm collision during surgery is tackled.

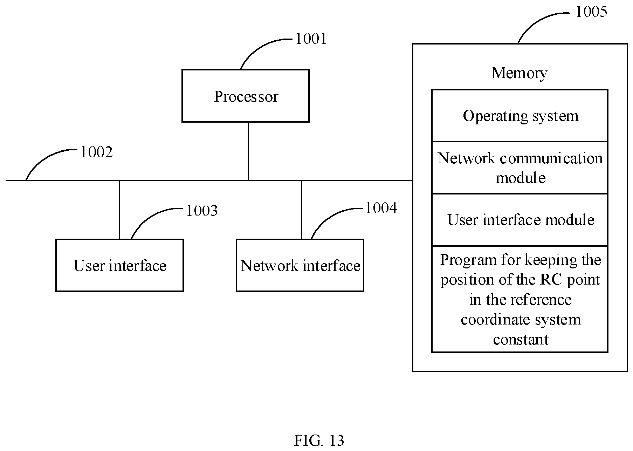

As shown in FIG. 13, which is a schematic diagram of a hardware structure involved in a robotic arm, a slave operating device, or a surgical robot for keeping a position of an RC point in a reference coordinate system ($F_0$) constant according to the embodiments of the present application. The robotic arm is taken as an example for illustration hereinbelow.

The robotic arm may include: a processor 1001, such as a CPU, a communication bus 1002, a user interface 1003, a network interface 1004, and a memory 1005. The communication bus 1002 is configured to realize connection and communication among these components. The user interface 1003 may include: a display, and an input unit, such as a keyboard. The network interface 1004 may optionally include a standard wired interface and a wireless interface (such as WI-FI interface). The memory 1005 may be a high-speed RAM memory, or a stable memory (non-volatile memory), such as a disk memory. Optionally, the memory 1005 may also be a storage device independent of the processor 1001 as described in the above.

Those skilled in the art can understand that the hardware structure as shown in FIG. 1 does not constitute a limitation to the image processing apparatus, and may include more or less components than what is shown in the figure, or combine some components, or adopt different arrangements of the components.

As shown in FIG. 13, as a computer storage medium, a memory 1005 may include: an operating system, a network communication module, a user interface module, and a program for keeping the position of the RC point in the reference coordinate system ($F_0$) constant.

In the robotic arm shown in FIG. 13, the network communication module is mainly configured to connect to a server and perform data communication with the server; and the processor 1001 may be configured to:

call the program for keeping the position of the RC point in the reference coordinate system constant, which is stored in the memory 1005, and to perform the following operations:

receiving a user input for performing adjustment to the cyclone joint;
controlling the cyclone joint to perform angle adjustment according to the user input;
calculating target positions of at least three joints of the adjustment arm according to the angle adjustment performed by the cyclone joint; and
controlling the at least three joints of the adjustment arm to perform position adjustment according to the target positions of the at least three joints so as to maintain the position of the RC point in the reference coordinate system constant.

Further, the processor 110 may be configured to call an image processing program of the endoscope stored in the memory 109, and to further perform the following operations:

obtaining the position of the RC point in the reference coordinate system ($F_0$) and a constant transformation ($T_{0a}$) from the reference coordinate system ($F_0$) to an adjustment arm coordinate system ($F_a$);
obtaining an angle value of the cyclone joint;
calculating a first transformation ($T_{bc}$) from a cyclone joint coordinate system ($F_b$) to an RC point coordinate system ($F_c$) based on the angle value of the cyclone joint;
calculating a second transformation ($T_{ab}$) from the adjustment arm coordinate system ($F_a$) to the cyclone joint coordinate system ($F_b$) based on the position of the RC point in the reference coordinate system ($F_0$), the constant transformation ($T_{0a}$), and the first transformation ($T_{bc}$); and
calculating the target positions of the at least three joints of the adjustment arm based on the second transformation ($T_{ab}$).

Further, the processor 110 may be configured to call the image processing program of the endoscope stored in the memory 109, and to further perform the following operations:

obtaining a third transformation ($T_{0c}$) from the reference coordinate system ($F_0$) to the RC point coordinate system ($F_c$) through a kinematic calculation; and
acquiring the position of the RC point in the reference coordinate system ($F_0$) according to the third transformation ($T_{0c}$).

Further, the processor 110 may be configured to call the image processing program of the endoscope stored in the memory 109, and to further perform the following operations:
obtaining the angle value of the cyclone joint from a joint encoder.
The joint encoder comprises a position sensor, and the position sensor is configured to measure an angle value of a joint where the joint encoder is located.
Further, the processor 110 may be configured to call the image processing program of the endoscope stored in the memory 109, and to further perform the following operations:
determining the first transformation ($T_{bc}$) from the cyclone joint coordinate system ($F_b$) to the RC point coordinate system ($F_c$) based on the angle value of the cyclone joint.
Further, the processor 110 may be configured to call the image processing program of the endoscope stored in the memory 109, and to further perform the following operations:

constructing a calculation model for the position of the RC point in the reference coordinate system ($F_0$) based on a coordinate transformation relationship; and
expanding the calculation model along an X-direction, a Y-direction, and a Z-direction to obtain three equations.

The equations comprise a multivariate linear equation system, in which the target positions of the at least three joints of the adjustment arm are used as unknown variables.
Further, the processor 110 may be configured to call the image processing program of the endoscope stored in the memory 109, and to further perform the following operations:

$$P_{0c} = R_{0a} * R_{ab} * P_{bc} + R_{0a} * P_{ab} + P_{0a},$$

in which,
$P_{0c}$ represents a position component of a transformation $T_{0c}$; $R_{0a}$ represents an attitude component of the transformation $T_{0a}$; $P_{0a}$ represents a position component of the transformation $T_{0a}$; $R_{ab}$ represents an attitude component of the transformation $T_{ab}$; $P_{ab}$ represents a position component of the transformation $T_{ab}$; $R_{bc}$ represents an attitude component of the transformation $T_{bc}$; and $P_{bc}$ represents a position component of the transformation $T_{bc}$.
Further, the processor 110 may be configured to call the image processing program of the endoscope stored in the memory 109, and to further perform the following operations:

expanding equation $P_{0c} = R_{0a} * R_{ab} * P_{bc} + R_{0a} * P_{ab} + P_{0a}$ according to position components in the X-direction, the Y-direction, and the Z-direction, respectively, to obtain a compensation solution model comprising the following equation system:

$$\begin{cases} P_x = f_1(\theta_1, \theta_2 \cdots \theta_i) \\ P_y = f_2(\theta_1, \theta_2 \cdots \theta_i) \\ P_z = f_3(\theta_1, \theta_2 \cdots \theta_i) \end{cases};$$

in which, $P_x$, $P_y$, and $P_z$ are three components of the RC point position $P_{0c}$ in the X-direction, the Y-direction, and the Z-direction, respectively, and $f_1$, $f_2$, and $f_3$ represent corresponding calculation functions, which are all related to positions $(\theta_1, \theta_2 \dots \theta_i)$ of the at least three joints of the adjustment arm.

Further, the processor 110 may be configured to call the image processing program of the endoscope stored in the memory 109, and to further perform the following operations:
based on that a position $\theta_1$ of the first rotary joint is a constant, obtaining target positions $(\theta_2, \theta_3, \theta_4)$ of the first linear joint, the second rotary joint, and the first linear joint according to the following compensation solution model:

$$\begin{cases} P_x = f_1(\theta_1, \theta_2, \theta_3, \theta_4) \\ P_y = f_2(\theta_1, \theta_2, \theta_3, \theta_4) \\ P_z = f_3(\theta_1, \theta_2, \theta_3, \theta_4) \end{cases}.$$

## Claims

1. A method for keeping a position of a remote center of manipulation (RC point) of a surgical robot constant in a reference coordinate system ($F_0$),

   the surgical robot comprising: a processor, an adjustment arm, and a cyclone joint; the adjustment arm comprising a plurality of joints; and the method being executed by a processor and comprising the following steps:

   receiving a user input for performing adjustment to the cyclone joint;
   controlling the cyclone joint to perform angle adjustment according to the user input;
   calculating target positions of at least three joints of the adjustment arm according to the angle adjustment performed by the cyclone joint; and
   controlling the at least three joints of the adjustment arm to perform position adjustment according to the target positions of the at least three joints so as to maintain the position of the RC point in the reference coordinate system constant.

2. The method according to claim 1, wherein the step of calculating the target positions of the at least three joints of the adjustment arm according to the angle adjustment performed by the cyclone joint comprises:

   obtaining the position of the RC point in the reference coordinate system ($F_0$) and a constant transformation ($T_{0a}$) from the reference coordinate system ($F_0$) to an adjustment arm coordinate system ($F_a$);
   obtaining an angle value of the cyclone joint;
   calculating a first transformation ($T_{bc}$) from a cyclone joint coordinate system ($F_b$) to an RC point coordinate system ($F_c$) based on the angle value of the cyclone joint;
   calculating a second transformation ($T_{ab}$) from the adjustment arm coordinate system ($F_a$) to the cyclone joint coordinate system ($F_b$) based on the position of the RC point in the reference coordinate system ($F_0$), the constant transformation ($T_{0a}$), and the first transformation ($T_{bc}$); and
   calculating the target positions of the at least three joints of the adjustment arm based on the second transformation ($T_{ab}$).

3. The method according to claim 2, wherein the step of obtaining the position of the RC point in the reference coordinate system ($F_0$) comprises:

   obtaining a third transformation ($T_{0c}$) from the reference coordinate system ($F_0$) to the RC point coordinate system ($F_c$) through a kinematic calculation; and
   acquiring the position of the RC point in the reference coordinate system ($F_0$) according to the third transformation ($T_{0c}$).

4. The method according to claim 2 or 3, wherein the step of obtaining the angle value of the cyclone joint comprises:

   obtaining the angle value of the cyclone joint from a joint encoder;
   wherein the joint encoder comprises a position sensor, and the position sensor is configured to measure an angle value of a joint where the joint encoder is located.

5. The method according to claim 2, wherein the step of calculating the first transformation ($T_{bc}$) from the cyclone joint coordinate system ($F_b$) to the RC point coordinate system ($F_c$) comprises:
   determining the first transformation ($T_{bc}$) from the cyclone joint coordinate system ($F_b$) to the RC point coordinate system ($F_c$) based on the angle value of the cyclone joint.

**6.** The method according to claim 2, wherein the step of calculating the second transformation ($T_{ab}$) from the adjustment arm coordinate system ($F_a$) to the cyclone joint coordinate system ($F_b$) comprises:

constructing a calculation model for the position of the RC point in the reference coordinate system ($F_0$) based on a coordinate transformation relationship; and

expanding the calculation model along an X-direction, a Y-direction, and a Z-direction to obtain three equations;

wherein
the equations comprise a multivariate linear equation system, in which the target positions of the at least three joints of the adjustment arm are used as unknown variables.

**7.** The method according to claim 6, wherein the step of constructing the calculation model for the position of the RC point in the reference coordinate system ($F_0$) based on the coordinate transformation relationship comprises:

$$P_{0c}=R_{0a}*R_{ab}*P_{bc}+ R_{0a}*P_{ab}+ P_{0a},$$

wherein

$P_{0c}$ represents a position component of a transformation $T_{0c}$;

$R_{0a}$ represents an attitude component of the transformation $T_{0a}$;

$P_{0a}$ represents a position component of the transformation $T_{0a}$;

$R_{ab}$ represents an attitude component of the transformation $T_{ab}$;

$P_{ab}$ represents a position component of the transformation $T_{ab}$;

$R_{bc}$ represents an attitude component of the transformation $T_{bc}$; and

$P_{bc}$ represents a position component of the transformation $T_{bc}$.

**8.** The method according to claim 7, wherein the step of expanding the calculation model along the X-direction, the Y-direction, and the Z-direction to obtain the three equations comprises:

expanding equation $P_{0c}=R_{0a}*R_{ab}*P_{bc}+R_{0a}*P_{ab}+P_{0a}$ according to position components in the X-direction, the Y-direction, and the Z-direction, respectively, to obtain a compensation solution model comprising the following equation system:

$$\begin{cases} P_x = f_1(\theta_1, \theta_2 \cdots \theta_i) \\ P_y = f_2(\theta_1, \theta_2 \cdots \theta_i) \\ P_z = f_3(\theta_1, \theta_2 \cdots \theta_i) \end{cases};$$

wherein, $P_x$, $P_y$, and $P_z$ are three components of the RC point position $P_{0c}$ in the X-direction, the Y-direction, and the Z-direction, respectively, and $f_1$, $f_2$, and $f_3$ represent corresponding calculation functions, which are all related to positions ($\theta_1, \theta_2 \dots \theta_i$) of the at least three joints of the adjustment arm.

**9.** The method according to claim 8, wherein the step of calculating target positions of at least three joints of the adjustment arm comprises: calculating the target positions of the at least three joints of the adjustment arm according to the compensation solution model.

**10.** The method according to claim 1, wherein the adjustment arm comprises: a first rotary joint, a first linear joint, a second rotary joint, and a second linear joint; or alternatively, the adjustment arm comprises: the first linear joint, the second rotary joint, and the second linear joint.

**11.** The method according to claim 10, wherein a position $\theta_1$ of the first rotary joint is a constant, and target positions ($\theta_2, \theta_3, \theta_4$) of the first linear joint, the second rotary joint, and the first linear joint are obtained according to the following compensation solution model:

$$\begin{cases} P_x = f_1(\theta_1, \theta_2, \theta_3, \theta_4) \\ P_y = f_2(\theta_1, \theta_2, \theta_3, \theta_4) \\ P_z = f_3(\theta_1, \theta_2, \theta_3, \theta_4) \end{cases}.$$

**12.** A robotic arm, in connection with an orientation platform, the robotic arm comprising:

an adjustment arm, in connection with the orientation platform, the adjustment arm comprising a plurality of joints;

a cyclone joint, in connection with the adjustment arm, with an axis passing through the cyclone joint being defined as a cyclone axis; and

a processor, in connection with the cyclone joint and the adjustment arm;

wherein
the processor is configured to execute the method according to any one of claims 1-11.

**13.** A slave operating device, comprising an orientation platform, wherein the slave operating device comprises the robotic arm according to claim 12, and the robotic arm is in connection with the orientation platform.

14. A surgical robot, comprising:

    the slave operating device according to claim 13; and
    a master operating device, configured to control the slave operating device.

15. A computer-readable storage medium, being stored with a program for keeping a position of an RC point in a reference coordinate system $(F_0)$ constant, which, when being executed by a processor, causes the processor to implement the steps of the method for keeping the position of the RC point in the reference coordinate system $(F_0)$ constant as described in the above.

1000

200

100

300

FIG. 1

FIG. 2

300

FIG. 3

FIG. 4

FIG. 5

FIG. 6

FIG. 7

FIG. 8

FIG. 9

$T_{0a}$ $F_a$ $F_0$ $T_{ab}$ $F_b$ $T_{bc}$ $F_c$

FIG. 10

| | |
|---|---|
| receiving a user input for performing adjustment to the cyclone joint | S10 |
| controlling the cyclone joint to perform angle adjustment according to the user input | S20 |
| calculating target positions of joints of the adjustment arm according to the angle adjustment performed by the cyclone joint | S30 |
| controlling at least three joints of the adjustment arm to perform position adjustment according to the target positions of the joints so as to maintain the position of the RC point in the reference coordinate system constant | S40 |

FIG. 11

EP 4 431 046 A1

```
┌─────────────────────────────┐
(  901: starting operation by the  )
(       cyclone joint              )
└─────────────────────────────┘
              │
┌─────────────────────────────┐
│  902: obtaining the position $P_{0c}$ of │
│  the RC point in the orientation │
│  platform coordinate system   │
└─────────────────────────────┘
              │
┌─────────────────────────────┐
│  903: adjusting an angle value of │ ◄───────┐
│  the cyclone joint according to the │      │
│            operation            │         │
└─────────────────────────────┘          │
              │                            │
┌─────────────────────────────┐          │
│  904: obtaining the angle value of │     │
│         the cyclone joint        │        │
└─────────────────────────────┘          │
              │                            │
┌─────────────────────────────┐          │
│  905: calculating the actual value of │   │
│    the transformation $T_{bc}$   │       │
└─────────────────────────────┘          │
              │                            │
┌─────────────────────────────┐          │
│  906: calculating the transformation │    │
│            $T_{ab}$              │         │
└─────────────────────────────┘          │
              │                            │
┌─────────────────────────────┐          │
│  907: calculating the target   │          │
│   positions of the joints of the │        │
│        adjustment arm           │         │
└─────────────────────────────┘          │
              │                            │
┌─────────────────────────────┐          │
│  908: adjusting positions of the │        │
│  joints according to the position │       │
│     compensation values         │         │
└─────────────────────────────┘          │
              │                            │
       ◇───────────────◇                  │
      ╱ 909: stopping operations ╲          │
      ╲                         ╱           │
       ◇───────────────◇                  │
   Yes  │        │ No                       │
        │        │                          │
        │   ◇─────────────────────◇        │
        │  ╱ 910: whether the cyclone joint or the joints of ╲  No
        │  ╲ the adjustment arm reach a limit ╱ ──────────────┘
        │   ◇─────────────────────◇
        │        │ Yes
        │        │
        │   ┌─────────────────────────────┐
        └──►(  911: stopping operations of the  )
            (        cyclone joint             )
            └─────────────────────────────┘
```

FIG. 12

22

FIG. 13

| INTERNATIONAL SEARCH REPORT | International application No. |
|---|---|
| | **PCT/CN2022/129288** |

**A. CLASSIFICATION OF SUBJECT MATTER**

A61B 34/30(2016.01)i

According to International Patent Classification (IPC) or to both national classification and IPC

**B. FIELDS SEARCHED**

Minimum documentation searched (classification system followed by classification symbols)

A61B

Documentation searched other than minimum documentation to the extent that such documents are included in the fields searched

Electronic data base consulted during the international search (name of data base and, where practicable, search terms used)

CNTXT; CNABS; ENTXT; ENTXTC; VEN: 不动点, 远程操纵中心, 远程中心, 关节, 角度, 保持; RC, RCM, remote center, joint?, articulation?, angle?, keep+, hold+, retain+.

**C. DOCUMENTS CONSIDERED TO BE RELEVANT**

| Category* | Citation of document, with indication, where appropriate, of the relevant passages | Relevant to claim No. |
|---|---|---|
| PX | CN 114129266 A (SHENZHEN JINGFENG MEDICAL SCIENCE AND TECHNOLOGY CO., LTD.) 04 March 2022 (2022-03-04)<br>claims 1-15 | 1-15 |
| PX | CN 114098954 A (SHENZHEN JINGFENG MEDICAL SCIENCE AND TECHNOLOGY CO., LTD.) 01 March 2022 (2022-03-01)<br>description, paragraphs 53-159, and figures 1-17 | 1-15 |
| X | CN 106028994 A (INTUITIVE SURGICAL OPERATIONS, INC.) 12 October 2016 (2016-10-12)<br>description, paragraphs 57-95, and figures 7-12 | 1-15 |
| A | CN 104546147 A (CHONGQING INSTITUTE OF GREEN AND INTELLIGENT TECHNOLOGY, CHINESE ACADEMY OF SCIENCES) 29 April 2015 (2015-04-29)<br>entire document | 1-15 |
| A | JP 2019098496 A (RIVERFIELD INC.) 24 June 2019 (2019-06-24)<br>entire document | 1-15 |
| A | WO 2017220722 A1 (KONINKLIJKE PHILIPS N.V.) 28 December 2017 (2017-12-28)<br>entire document | 1-15 |

☐ Further documents are listed in the continuation of Box C.   ☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **27 December 2022** | **28 January 2023** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**
Information on patent family members

International application No.

**PCT/CN2022/129288**

| Patent document cited in search report | | | Publication date (day/month/year) | Patent family member(s) | | | Publication date (day/month/year) |
|---|---|---|---|---|---|---|---|
| CN | 114129266 | A | 04 March 2022 | None | | | |
| CN | 114098954 | A | 01 March 2022 | None | | | |
| CN | 106028994 | A | 12 October 2016 | US | 2021267699 | A1 | 02 September 2021 |
| | | | | JP | 2017513539 | A | 01 June 2017 |
| | | | | US | 2017181801 | A1 | 29 June 2017 |
| | | | | US | 2019209251 | A1 | 11 July 2019 |
| | | | | EP | 3107478 | A1 | 28 December 2016 |
| | | | | WO | 2015127078 | A1 | 27 August 2015 |
| | | | | KR | 20160124112 | A | 26 October 2016 |
| | | | | CN | 106028994 | B | 22 January 2019 |
| | | | | EP | 3107478 | A4 | 18 October 2017 |
| | | | | US | 10285764 | B2 | 14 May 2019 |
| | | | | JP | 6559691 | B2 | 14 August 2019 |
| | | | | EP | 3107478 | B1 | 09 December 2020 |
| | | | | US | 11033345 | B2 | 15 June 2021 |
| | | | | KR | 102327850 | B1 | 17 November 2021 |
| CN | 104546147 | A | 29 April 2015 | None | | | |
| JP | 2019098496 | A | 24 June 2019 | None | | | |
| WO | 2017220722 | A1 | 28 December 2017 | EP | 3474763 | A1 | 01 May 2019 |
| | | | | US | 2019175293 | A1 | 13 June 2019 |
| | | | | JP | 2019525787 | A | 12 September 2019 |

Form PCT/ISA/210 (patent family annex) (January 2015)

**EP 4 431 046 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202111334158